(19) Europäisches Patentamt
European Patent Office
Office européen des brevets

(11) EP 1 975 142 A1

(12) EUROPEAN PATENT APPLICATION
published in accordance with Art. 158(3) EPC

(43) Date of publication:
01.10.2008 Bulletin 2008/40

(21) Application number: 06832615.6

(22) Date of filing: 14.11.2006

(51) Int Cl.:
*C07B 61/00* (2006.01) *C07D 403/14* (2006.01)
*C07D 409/14* (2006.01)

(86) International application number:
PCT/JP2006/322658

(87) International publication number:
WO 2007/060860 (31.05.2007 Gazette 2007/22)

(84) Designated Contracting States:
AT BE BG CH CY CZ DE DK EE ES FI FR GB GR HU IE IS IT LI LT LU LV MC NL PL PT RO SE SI SK TR

(30) Priority: 22.11.2005 JP 2005336811

(71) Applicants:
- Nihon University
Tokyo 102-8275 (JP)
- Gentier Biosystems, Inc
Chiyoda-ku
Tokyo
102-0076 (JP)

(72) Inventors:
- SUGIYAMA, Hiroshi
Kyoto-shi, Kyoto 6028442 (JP)
- DOHNO, Chikara
Ibaraki-shi, Osaka 5670872 (JP)
- FUKUDA, Noboru
Tokyo 1028275 (JP)

(74) Representative: Forstmeyer, Dietmar et al
BOETERS & LIECK
Oberanger 32
80331 München (DE)

# (54) AUTOMATED SOLID PHASE SYNTHESIS OF PYRROLE-IMIDAZOLE POLYAMIDE

(57) [PROBLEMS]

To provide a synthesis method for pyrrole-imidazole polyamide which is automated at a higher level and can produce a product with a high yield in a more stable manner.

[MEANS FOR SOLVING PROBLEMS]

In a solid phase synthesis method, the automated synthesis of a polyimide can be promoted by using HCTU as a condensation-activator, the yield of a product can be increased by charging a monomer in a solid form, and a pyrrole-imidazole polyamide having any sequence can be synthesis by combining the synthesis method with a manual synthesis with an acid chloride.

EP 1 975 142 A1

## Description

Technical Field

Conventionally, pyrrole-imidazole has been synthesized by a liquid-phase method or by an automated synthesis method based on a solid-phase Fmoc method using a peptide synthesizer using HATU (0-[7-azobenzotriazol-1-yl]-1,1,3,3-tetramethyluronium hexafluorophosphate), HBTU (2-[1H-benzotriazol-1-yl]-1,1,3,3-tetramethyluronium hexafluorophosphate), or the like as a condensation-activator (see, Patent Document 1, for example). In addition, modified solid-phase peptide synthesis has been developed, which has such a feature that the C-terminal part added to a solid phase is suitably protected during a coupling step, and includes a pre-sequence including specific amino acids (see, Patent Document 2, for example).

However, those methods each require a high technology, a complicated operation, and a long producing period. Moreover, those methods have not achieved fully automated synthesis because those methods include many steps requiring manual procedures, and have difficulty in synthesizing an imidazole-pyrrole sequence. Therefore, it is desired to provide a method of synthesizing a pyrrole-imidazole polyamide, which is automated at a higher level and can easily synthesize a pyrrole-imidazole polyamide having any sequence with a higher yield in a more stable manner compared with the conventional methods.

Patent Document 1: WO 2003/000683

Patent Document 2: JP 2001-500134 A

Disclosure of the Invention

Problems to be solved by the Invention

It is an object of the present invention to provide a method of synthesizing a pyrrole-imidazole polyamide, which is automated at a higher level and can produce a product with a high yield in a more stable manner.

Means for solving the Problems

The inventors of the present invention have made extensive studies to solve the above-mentioned problems. As a result, the inventors have found that automated synthesis of a polyamide can be promoted by using HCTU as a condensation-activator in the solid-phase synthesis method. In addition, the inventors have found that the yield of a pyrrole-imidazole polyamide can be increased by charging a material monomer in a solid form.
The inventors have further found that a pyrrole-imidazole polyamide having any sequence can be synthesized by combining the automated synthesis with a manual synthesis using an acid chloride, thus completing the present invention. Moreover, the inventors have found that the completely automated synthesis of a pyrrole-imidazole polyamide can be achieved by using pyrrole-imidazole dimer as part of materials.

That is, the present invention relates to the following items (1) to (10):

(1) a method of synthesizing a pyrrole-imidazole polyamide by a solid-phase synthesis method, including using HCTU (1-[bis(dimethylamino)methylene] -5-chloro-1H-benzotriazolium 3-oxide hexafluorophosphate) as a condensation-activator;
(2) a synthesis method according to Item (1), including charging a material monomer in a solid form;
(3) a synthesis method according to Item (1) or (2), including forming a pyrrole bond or an imidazole bond to an N-terminal by an acid chloride method;
(4) a synthesis method according to Item (3), in which the material monomer is a pyrrole acid chloride or an imidazole acid chloride;
(5) a synthesis method according to Item (1), including charging a pyrrole material and an imidazole material as a pyrrole-imidazole dimer based on a dimer method;
(6) a synthesis method according to Item (5), which is performed by automated synthesis;
(7) a pyrrole-imidazole polyamide, which is represented by Formula 1;

[Formula 1]

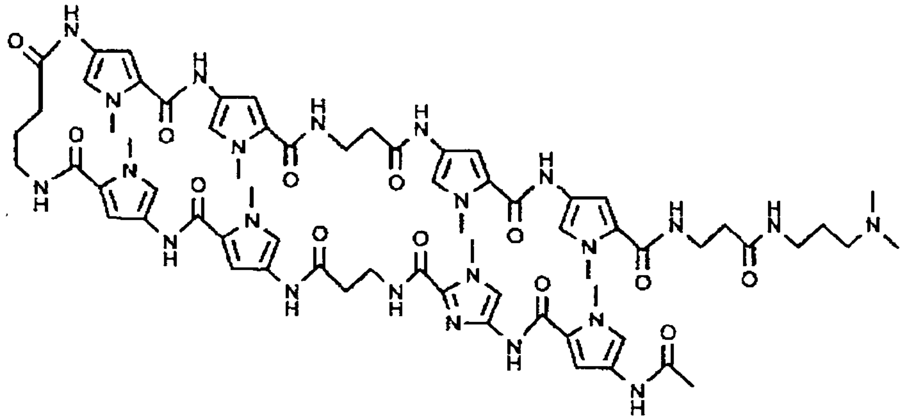

(8) a pyrrole-imidazole polyamide, which is represented by Formula 2;

[Formula 2]

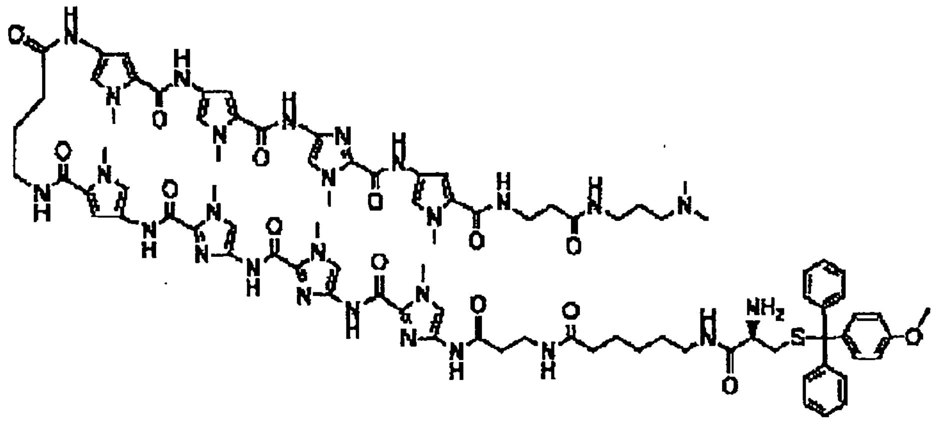

(9) a pyrrole-imidazole polyamide, which is represented by Formula 3; and

[Formula 3]

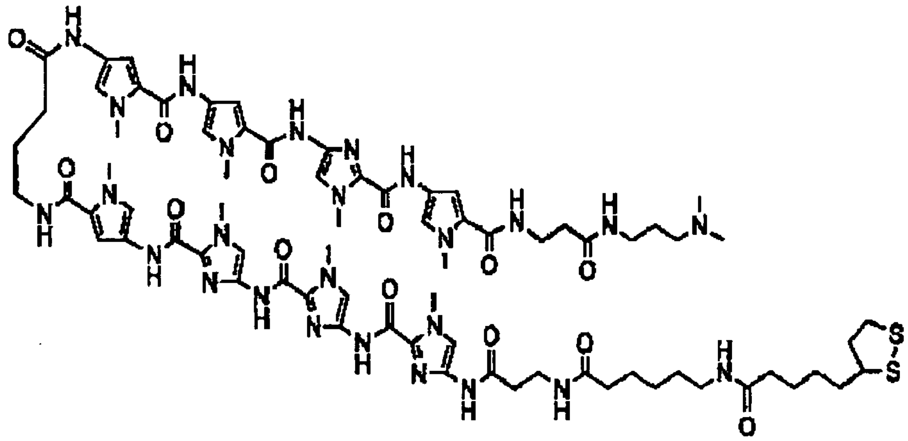

(10) a pyrrole-imidazole polyamide, which is represented by Formula 4.

[Formula 4]

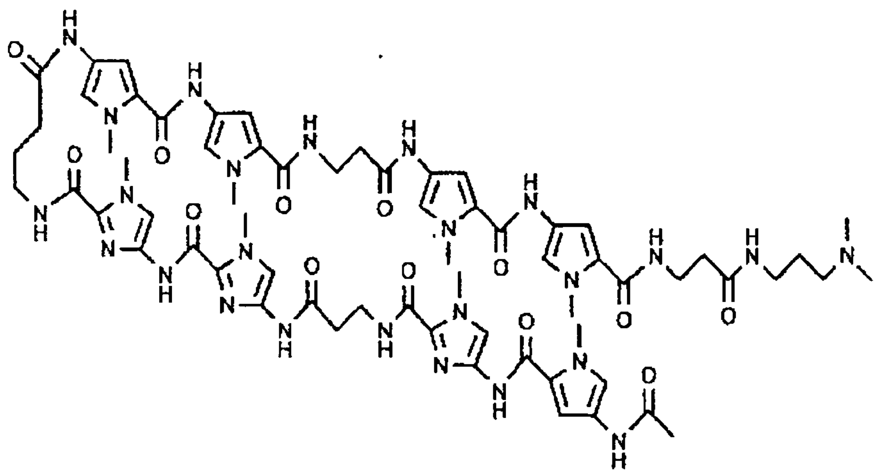

Effect of the Invention

According to the synthesis method of the present invention, it is possible to synthesize a pyrrole-imidazole polyamide rapidly and quantitatively. The synthesized pyrrole-imidazole polyamide is useful for medical purposes or the like because the pyrrole-imidazole polyamide can recognize and specifically bond to a DNA sequence. Moreover, if the synthesis method is incorporated in a system, it is possible to provide a synthesizer that is automated at a higher level and can produce a product with a high yield in a more stable manner.

Brief Description of the Drawings

Fig. 1 is a chromatogram showing the result of HPLC after separation/purification of AcPyImβPyPyγPyPyβPyβDp (Example 1).

Fig. 2 is a chromatogram showing the result of HPLC after separation/purification of AcPyImβImImγPyPyβPyPyβDp (Example 12).

Best Mode for carrying out the Invention

The synthesis method of the present invention may be any known solid-phase synthesis method such as the Boc method or the Fmoc method as long as HCTU is used as a condensation-activator. Of those, the Fmoc method is preferably employed.

The synthesis method of the present invention can be performed by repeating a condensation reaction of a protected amino acid, a deprotection reaction of an $N^{\alpha}$-amino-protected group, etc. on an insoluble solid-phase carrier.

The synthesis method of the present invention includes: a synthesis method performed by automated synthesis in combination with manual synthesis; and a synthesis method performed by only automated synthesis. In the synthesis method performed by automated synthesis in combination with manual synthesis, the manual synthesis is preferably performed by the acid chloride method. According to the acid chloride method, any pyrrole-imidazole polyamide can be synthesized because the coupling yield of pyrrole and imidazole reaches 90% or more.

A pyrrole acid chloride or an imidazole acid chloride to be used in the acid chloride method may be synthesized by any method. The pyrrole acid chloride and imidazole acid chloride are particularly preferably compounds synthesized by the methods described in examples of the present invention.

On the other hand, in the synthesis method performed by only automated synthesis, the dimer method is preferably employed. The dimer method enables fully automated synthesis of a compound of interest, i.e., a pyrrole-imidazole polyamide.

In the case where the synthesis method of the present invention is performed by automated synthesis, an automated synthesizer is preferably used. The automated synthesizer may be any known apparatus such as a semi-automated synthesizer, a fully-automated synthesizer, or a multipeptide synthesizer, and it is particularly preferably a peptide synthesizer.

A monomer to be used for synthesis of the present invention may be a commercially-available monomer such as FmocPyCOOH (manufactured by Wako Pure Chemicals Industries, Ltd.) or FmocImCOOH (manufactured by Wako Pure Chemicals Industries, Ltd.). In addition, for some compounds to be synthesized, the monomer may be Fmoc-γ-Abu-OH (manufactured by Novabiochem), Fmoc-β-Ala-OH (manufactured by Novabiochem), etc. If Fmoc-β-Ala-OH or Fmoc-γ-Abu-OH is integrated into the structure of a pyrrole-imidazole polyamide to be synthesized, the distortion of a compound to be produced can be avoided, resulting in improved affinity to a DNA.

Meanwhile, FmocPyImCOOH dimer (manufactured by Wako Pure Chemicals Industries, Ltd.) or the like may be used as a dimer. The monomer, dimer, etc. to be used for synthesis are not particularly limited as long as they can be used in synthesis.

In the case where a monomer is used in the synthesis method of the present invention, it is preferable to introduce the monomer in a solid form. The term "to introduce the monomer" refers to introduction of a monomer into a synthesis process, which includes placing a monomer in a test tube on a rack of a synthesizer.

In a conventional method, a monomer is dissolved in a solvent such as DMF and introduced in a liquid form into a synthesis process. However, in this method, a monomer is easily decomposed because the monomer is retained in a liquid state, resulting in a low yield of a pyrrole-imidazole polyamide synthesized.

On the other hand, introduction of a monomer in a solid form is preferable because the monomer is hardly decomposed, resulting in a high yield of a pyrrole-imidazole polyamide synthesized. The phrase "introduction of a monomer of the present invention in a solid form" refers to a procedure including addition of a monomer to a test tube and introduction of the monomer into a synthesis process without further treatment.

Before introduction of a monomer into a solid form, the monomer is preferably ground by a pestle and mortar. In addition, the monomer is preferably dried well under reduced pressure just before the synthesis. Those treatments can increase the yield of a pyrrole-imidazole polyamide synthesized.

Hereinafter, the present invention will be described in more detail by way of examples, which should not be construed as limiting the invention thereto.

[Example 1]

<Synthesis of pyrrole-imidazole polyamide using HCTU (acid chloride method)>

AcPyImβPyPyγPyPyβPyPyβDp (Formula 1) was synthesized using HCTU as a condensation-activator by combining automated synthesis with manual synthesis based on the acid chloride method. The abbreviations in Formulae have the following means (Ac: acetyl, Py: pyrrole, Im: imidazole, β: β-alanine, γ: γ-butyric acid, Dp:

N,N-dimethyl-1,3-propanediamine).

[Formula 1]

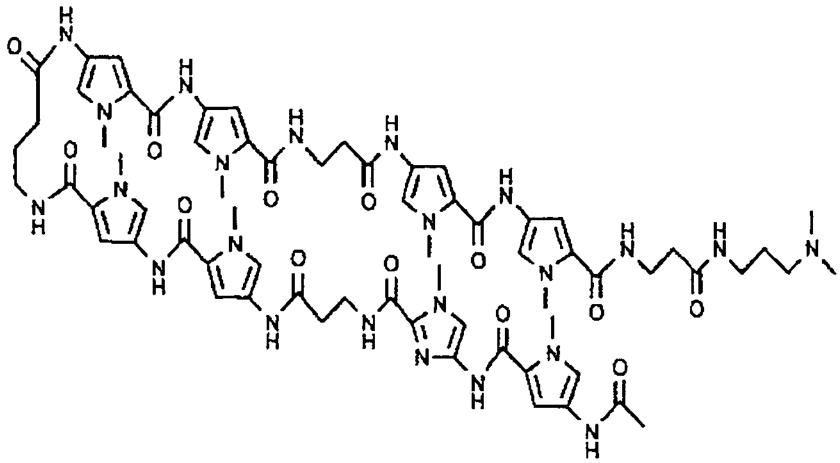

1. Preparation of reagent

1) Monomer

FmocPyCOOH (Wako, 145 mg), FmocImCOOH (Wako, 145 mg), Fmoc-γ-Abu-OH (Novabiochem, 130 mg), and Fmoc-β-Ala-OH (Novabiochem, 125 mg) were prepared in amounts for seven couplings, one coupling, one coupling, and three couplings, respectively, and the monomers were separately weighed in an amount of four equivalents with respect to a resin and transferred to test tubes. The weighed monomers were ground by a pestle and mortar and dried under reduced pressure.

2) Reagent for synthesis

The reagents described in Table 1 were prepared and used for synthesis by a synthesizer.
[Table 1]

| | |
|---|---|
| Activator 1 Condensation reagent | HCTU/DMF(N,N-Dimethylformamide) (0.5M) |
| | HCTU (manufactured by Peptide Institute, Inc.) 10.34g/DMF (manufactured by Wako Pure Chemicals Industries, Ltd.) 50mL |
| | prepared by dissolving HCTU (PEPTIDE INSTITUTE Inc.) in anhydrous DMF in the nitrogen atmosphere. |
| Activator 2 Condensation reagent | DIEA (N,N-Diisopropylethylamine)/DMF(1M) |
| | DIEA (manufactured by Nacalai Tesque, Inc.) 16mL+DMF 84mL |
| Auxilliary 1 Reagent for washing of resin after synthesis | MeOH |
| Auxilliary 3 Capping agent | $Ac_2O$/pyridine/DMF (1:1:18) |
| | $Ac_2O$ 30mL+Pyridine 30mL+DMF 270mL |
| Deblock Reagent for deprotection of Fmoc group | 20% Piperidine/DMF |
| | Piperidine 200mL+DMF 800mL |
| Wash Solvent for washing | DMF |
| | DMF for peptide synthesis |

2. Preparation of column

Fmoc-β-Ala-CLEAR-Acid-Resin (manufactured by Peptide Institute, Inc.) was separated in an amount of 200 mg (0.1 mmol) and allowed to swell in 2 mL of DMF for 30 minutes. The swollen resin was transferred together with DMF to a column, and the column was prepared so that it could be used in a synthesizer without further treatments.

3. Peptide synthesis (Automated)

The 0.5 M HCTU/DMF and 1 M DIEA/DMF, prepared in the section 1. above, were installed as condensation-activators in a synthesizer (peptide synthesizer PIONEER; Applied Biosystems Japan), and the above-mentioned test tubes containing the respective monomers were arranged in order from the C-terminal in racks in the synthesizer.
The monomers in the respective test tubes arranged in racks in the synthesizer were dissolved in DMF, and the synthesizer was started to perform automated synthesis of $H_2$NImβPyPyγPyPyβPyPyβ-Resin by repeating a reaction cycle of the following items (1) to (3). The resultant $H_2$NImβPyPyγPyPyβPyPyβ-Resin was allowed to swell in 2 mL of DMF for 30 minutes while an FmocPy acid chloride to be used in the subsequent manual synthesis was prepared.

(1) A coupling treatment was performed in DMF using the above-mentioned activators for 1 hour.
(2) An acetyl capping treatment ($Ac_2O$: pyridine:DMF = 1:1:18) was performed to stop elongation of miscoupled products.
(3) After Fmoc deprotection (20% piperidine/DMF), the step (1) was performed again for the next coupling reaction. This cycle was repeated until a target product was produced.

4. Peptide synthesis (manual: acid chloride method)

In the nitrogen atmosphere, FmocPyCOOH (145 mg) was suspended in an amount for one coupling in anhydrous methylene chloride (manufactured by Nacalai, 2 mL). Oxalyl chloride (manufactured by Nacalai Tesque, Inc., 52 μL) was added dropwise to the suspension, and DMF (2 μL) was added, followed by stirring at room temperature for 30 minutes. After the reaction, the solvent was distilled off under reduced pressure, to thereby yield an FmocPy acid chloride as a foam-like solid.
Pyridine (130 μL) was added to the swollen $H_2$NImβPyPyγPyPyβPyPyβ-Resin, and the FmocPy acid chloride dissolved in methylene chloride (1 mL) was added thereto. The whole was shaken at room temperature for 15 minutes, and the resin was washed well with DMF, methanol, and methylene chloride. The resultant resin was allowed to swell in DMF and installed in the synthesizer again, followed by an acetyl capping treatment.

5. Purification

The resin was taken out from the synthesizer, and it was washed and dried. Then, N,N-dimethylpropanediamine (manufactured by Nacalai Tesque, Inc., 2 mL) was added thereto, and the whole was stirred well at 55°C for 10 hours, followed by cleavage of a polyamide from the resin. The resin was removed by filtration using Celite, and a polyamide was recovered with a solution of methanol: methylene chloride = 1:1.
The solvent was distilled off, and the polyamide was dissolved in DMF again, followed by separation/purification by HPLC. The result of HPLC after the separation/purification is shown in Fig. 1 (0.1% AcOH: $CH_3CN$ = 100:0 to 33.3:66.7, 20 min). After separation/purification, the resultant product was freeze-dried, to thereby yield a target polyamide AcPy-ImβPyPyγPyPyβPyPyβDp (53.5 mg, 37.6%, ESI mass found; 1423.0, calcd for $[M+H]^+$; 1422.7).

[Examples 2 to 7]

Pyrrole-imidazole polyamides described in Table 2 were synthesized in the same way as Example 1, and the yields of the compounds were determined. The structural formula of the pyrrole-imidazole polyamide synthesized in Example 4 is shown in Formula 2, and the structural formula of the pyrrole-imidazole polyamide synthesized in Example 6 is shown in Formula 3.
[Table 2]

| Example | Compound | Recovered product weight | Yield |
|---|---|---|---|
| 2 | AcImPyβPyγImβPyPyβDp | 45.3 mg | 38.5% |

(continued)

| Example | Compound | Recovered product weight | Yield |
|---|---|---|---|
| 3 | AcImPyPyβImPyPyγPyPyPyβPyPyPyβDp | 67.8 mg | 35.5% |
| 4 | MMt-CysεβImImImPyγPyPyPyPyβDp (Formula 2) | 67.3 mg | 37.4% |
| 5 | AcPyβImImβImImγPyPyβPyPyβImβDp | 68.6 mg | 37.9% |
| 6 | DTP-···ImImImPyγPyPyPyPyβDp (Formula 3) | 67.3 mg | 37.4% |
| 7 | AcPyPyPyβImPyγPyPyβIPyPyPyβDp | 58.0 mg | 34.8% |

[Formula 2]

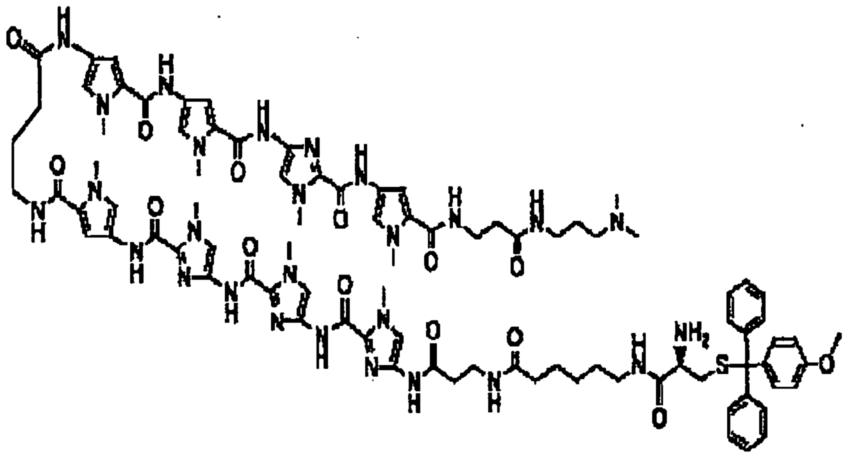

[Formula 3]

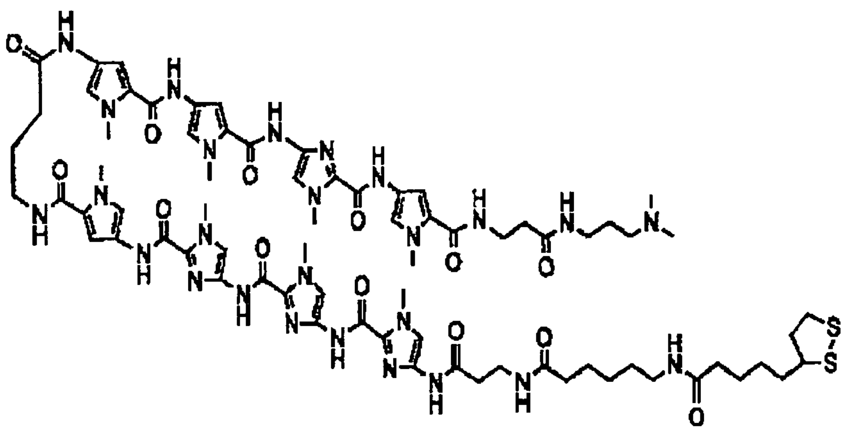

[Comparative Example 1]

<Synthesis of pyrrole-imidazole polyamide using HBTU>

The compound synthesized in Example 2 above (AcImPyβPyγImβPyPyβDp) was synthesized using HBTU as a condensation-activator in the same way as Example 1.
The same monomers, reagents for synthesis, and column as those in Example 1 were used to perform synthesis of a polyamide using a synthesizer, which includes manual synthesis based on the acid chloride method. Although HCTU was used as a condensation-activator in the method of Example 1, HBTU was used in this case, and HBTU/DMF (0.5 M) was used as Activator 1 condensation reagent.
The amount of the resultant pyrrole-imidazole polyamide is very small, and it was impossible to determine the yield of the compound in an appropriate manner. Those results reveal that a pyrrole-imidazole polyamide cannot be fully synthesized by the acid chloride method using HBTU as a condensation-activator.

[Comparative Example 2]

<Synthesis of pyrrole-imidazole polyamide using HATU>

Pyrrole-imidazole polyamides described in Table 3 were synthesized using HATU as a condensation-activator by combining automated synthesis with manual synthesis based on the acid chloride method, and the yields of the compounds were determined.
The same monomers, reagents for synthesis, and column as those in Example 1 were used to perform synthesis of a polyamide using a synthesizer, which includes manual synthesis based on the acid chloride method. In the method of Example 1, the monomers were introduced in solid forms, but in this case, the monomers were dissolved in DMF before introducing the monomers, and the monomers were introduced by a general method, i.e., charged in liquid forms. The recovered product weights of pyrrole-imidazole polyamides in the case of introducing the monomers in liquid forms were measured in the same way as Example 1, and the yields were calculated. The weights and yields are shown in Table 3.
As a result, it was verified that, in the case of using HATU as a condensation-activator and introducing a monomer in a liquid form, the yield was low (less than 10%), while in the case of using HCTU as a condensation-activator and introducing a monomer in a solid form, the yield was raised to 20 to 40%.
[Table 3]

| Compound | Condensation-activator | Monomer introduction | Recovered product weight | Yield |
|---|---|---|---|---|
| AcImPyPyPyβDp | HATU | Liquid | 2.4 mg | 3.4% |
| AcImImPyPyβDp | | | 5.3 mg | 7.5% |

[Example 8]

<Synthesis of pyrrole-imidazole polyamide using HCTU>

AcPyPypImPyPyγImPyPyβImImβDp was synthesized using HCTU as a condensation-activator in the same way as Example 1, and the yield was determined. The recovered product weight and yield are shown in Table 4.

[Comparative Example 3]

<Synthesis of pyrrole-imidazole polyamide using HATU>

AcPyPyβImPyPyγImPyPyβImImβDp was synthesized using HATU as a condensation-activator in the same way as Example 8, and the yield was determined. The recovered product weight and yield are shown in Table 4.
[Table 4]

| | Compound | Condensation-activator | Recovered product weight | Yield |
|---|---|---|---|---|
| Example 8 | AcPyPyβImPyPyγIm PyPyβImImβDp | HCTU | 56.9 mg | 34.1% |
| Comparative Example 3 | | HATU | 41.1 mg | 24.6% |

The results of Example 8 and Comparative Example 3 reveal that the yield of the compound in the case of using HCTU as a condensation-activator is higher than that in the case of using HATU.

[Example 9]

<Synthesis of pyrrole-imidazole polyamide by charging monomer in solid form>

AcPyPyβImPyPyγImPyPyβImImβDp was synthesized using HCTU as a condensation-activator in the same way as Example 1, and the yield was determined. In this synthesis, the monomer was introduced in a solid form. The recovered product weight and yield are shown in Table 5.

[Comparative Example 4]

<Synthesis of pyrrole-imidazole polyamide by introducing monomer in liquid form>

AcPyPyβImPyPyγImPyPyβImImβDp was synthesized using HCTU as a condensation-activator in the same way as Example 9, and the yield was determined. In this synthesis, the monomer was dissolved in DMF and charged by a general method, i.e., introduced in a liquid form. The recovered product weight and yield are shown in Table 5.
[Table 5]

| | Compound | Introduction of monomer | Recovered product weight | Yield |
|---|---|---|---|---|
| Example 9 | AcPyPyβImPyPyγIm PyPyβImImβDp | Solid | 56.9 mg | 34.1% |
| Comparative Example 4 | | Liquid | 15.8 mg | 9.5% |

The results of Example 9 and Comparative Example 4 reveal that the yield of the compound in the case of introducing the monomer in a solid form is higher than that in the case of introducing the monomer in a liquid form.

[Example 10]

<Study on coupling yield>

Coupling was performed by the acid chloride method. In the same way as Example 1, a column was prepared using Fmoc-β-Ala-CLEAR-Acid-Resin (manufactured by Peptide Institute, Inc.). Then, 0.5 M HCTU/DMF and 1 M DIEA/DMF, prepared as described in Table 1, were used to automatically synthesize a resin having an $H_2$NIm terminal (manufactured by Peptide Institute, Inc.), $H_2$NImPy-Resin, using a synthesizer (peptide synthesizer PIONEER; Applied Biosystems Japan). The resultant $H_2$NImPy-Resin was allowed to swell in 2 mL of DMF for 30 minutes while an FmocPy acid chloride to be used in the subsequent manual synthesis was prepared.
In the nitrogen atmosphere, FmocPyCOOH (90.6 mg) was suspended in anhydrous methylene chloride (manufactured by Nacalai, 2 mL). Oxalyl chloride (manufactured by Nacalai Tesque, Inc., 34 μL) was added dropwise to the suspension, and DMF (2 μL) was added thereto, followed by stirring at room temperature for 30 minutes. After the reaction, the solvent was distilled off under reduced pressure, to thereby yield an FmocPy acid chloride as a foam-like solid.
The $H_2$NmPy-Resin prepared above was allowed to swell in methylene chloride (600 μL), and pyridine (130 μL) was added thereto, followed by addition of the above-prepared FmocPy acid chloride. The whole was shaken at room temperature for 1 hour, and the resin was washed well with DMF, methanol, and methylene chloride.
In the same way as Example 1, separation/purification and freeze-drying were performed, to thereby yield a pyrrole-imidazole polyamide. The resultant pyrrole-imidazole polyamide was subjected to HPLC measurement and weight measurement, and the coupling yield was calculated.
As shown in Formula 1, the coupling yield was calculated by comparing a peak area of a coupled compound with a peak area of an uncoupled compound by HPLC.
[Formula 1]

(Peak area of coupled compound/Molar absorption coefficient)/ (Peak area of uncoupled compound/Molar absorption coefficient + Peak area of coupled compound/Molar absorption coefficient) × 100

[Comparative Example 5]

<Study on coupling yield>

Coupling was performed by the acid anhydride method. A resin having an $H_2NIm$ terminal was allowed to swell in 2 mL of DMF for 30 minutes.
In the nitrogen atmosphere, FmocPyCOOH (362.4 mg) and DCC (N,N-dicyclohexylcarbodiimide; TCI, 206.3 mg) were suspended in anhydrous methylene chloride (manufactured by Nacalai, 1.5 mL), and the whole was stirred at room temperature for 1 hour. DMAP (4-dimethylaminopyridine; Wako) (61 mg) was added thereto, and the suspension was further stirred for 3 minutes.
The above-mentioned suspension was added to the swollen resin, and the whole was shaken at room temperature for 1 hour, followed by washing of the resin with DMF, methanol, and methylene chloride.
In the same way as Example 1, separation/purification and freeze-drying were performed, to thereby yield a pyrrole-imidazole polyamide. The resultant pyrrole-imidazole polyamide was subjected to HPLC measurement and weight measurement, and a coupling yield was calculated in the same way as Example 9.
The coupling yields calculated in Example 9 and Comparative Example 5 are shown in Table 6. The results reveal that the coupling yield in the case of the acid chloride method is higher than that in the case of the acid anhydride method.
[Table 6]

| | Coupling method | Yield (%) |
|---|---|---|
| Example 10 | Acid chloride method | 93% |
| Comparative Example 5 | Acid anhydride method | 79% |

[Comparative Example 6]

<Synthesis of pyrrole-imidazole polyamide by acid anhydride method>

Pyrrole-imidazole polyamides, AcImImβImPyImγPyPyPyβPyPyβDp and AcPyPyPyβImPyγPyImβImPyImβDp, were synthesized using HCTU as a condensation-activator by combining automated synthesis with manual synthesis based on the acid anhydride method.
The same monomers, reagents for synthesis, and column as those in Comparative Example 5 were used to perform synthesis of a polyamide using a synthesizer, which includes manual synthesis by the acid anhydride method. In the synthesis of AcImImβImPyImγPyPyPyβPyPyβ, it was impossible to synthesize the product and difficult to perform quantitative ImPy coupling. On the other hand, in the synthesis of AcPyPyPy-ImPyγPy-ImPyImβDp, it was impossible to synthesize the product because the Im → Py immediately subsequent to the resin was not achieved at all. Therefore, it was found that a pyrrole-imidazole polyamide could not be synthesized sufficiently by the acid anhydrous method.

[Example 11]

<Synthesis of pyrrole-imidazole polyamide using HCTU (dimer method)>

AcPyPyPyβImPyγImPyβPyPyImβDp was synthesized by automated synthesis based on the dimer method (manual method).

1. Preparation of reagent

1) Dimer

FmocPyImCOOH dimer (manufactured by Wako Pure Chemicals Industries, Ltd., 194 mg) was used in an amount for one coupling. Meanwhile, Fmoc-γ-Abu-OH (Novabiochem, 130 mg), Fmoc-β-Ala-OH (Novabiochem, 125 mg), FmocPyCOOH (Wako, 145 mg), and FmocImCOOH (Wako, 145 mg) were used in amounts for one coupling, two couplings, six couplings, and couplings, respectively.
The dimers were weighed in an amount of four equivalents with respect to a resin and transferred to test tubes. The dimers were dried under reduced pressure and dissolved in 2 mL of DMF.

2) Reagent for synthesis

The reagents described in Table 1 were prepared and used.

2. Preparation of column

In the same way as Example 1, a column was prepared using Fmoc-β-Ala-CLEAR-Acid-Resin (manufactured by Peptide Institute, Inc.).

3. Peptide synthesis

0.5 M HCTU/DMF and 1 M DIEA/DMF, prepared as described in Table 1, were used to automatically synthesize a resin having an $H_2$NIm terminal (manufactured by Peptide Institute, Inc.), $H_2$NβPyPyγPyPyβPyPyβ-Resin using a synthesizer (peptide synthesizer PIONEER; Applied Biosystems Japan). The resultant $H_2$NβPyPyγPyPyβPyPyβ-Resin was allowed to swell in 2 mL of DMF for 30 minutes while an activated ester of the FmocPyImCOOH dimer to be used in the subsequent automated synthesis-2 was prepared.

5. Peptide synthesis

FmocPyImCOOH (325 mg) and HOBt (HOBt:butanol, manufactured by Novabiochem, 147 mg) were weighed and added to a centrifuge tube and dissolved in DMF (1 mL). DCC (manufactured by TCI) (246 mg) was added thereto, and the whole was allowed to stand at room temperature for about 10 minutes, to thereby yield an activated ester of the FmocPyImCOOH dimer.
The swollen $H_2$NβPyPyγPyPyβPyPyβ-Resin was added together with DMF, and DIEA (manufactured by Nacalai Tesque, Inc., 650 μL) was added thereto, followed by shaking at room temperature for 12 hours. After the reaction, the resin was washed well with DMF, methanol, and methylene chloride.

5. Purification

The resin was taken out from the synthesizer, and it was washed and dried. Then, DCC (manufactured by Nacalai Tesque, Inc., 2 mL) was added thereto, and the whole was stirred well at 55°C for 10 hours, followed by cleavage of a polyamide from the resin. The resin was removed by filtration using Celite, and a polyamide was recovered with a solution of methanol: methylene chloride = 1:1.
The solvent was distilled off, and the polyamide was dissolved in DMF again, followed by separation/purification by HPLC. After separation/purification, the resultant product was freeze-dried, to thereby yield AcPyPyPyβImPyγImPyβ PyPyImβDp (72.1 mg, 43.2%, ESI mass found; 1667.0, calcd for $[M+H]^+$; 1666.8).

[Example 12]

<Synthesis of AcPyImβImImγPyPyβPyPyβDp by acid chloride method>

AcPyImβImImγPyPyβPyPyβDp (Formula 4) was synthesized in the same way as Example 1.

[Formula 4]

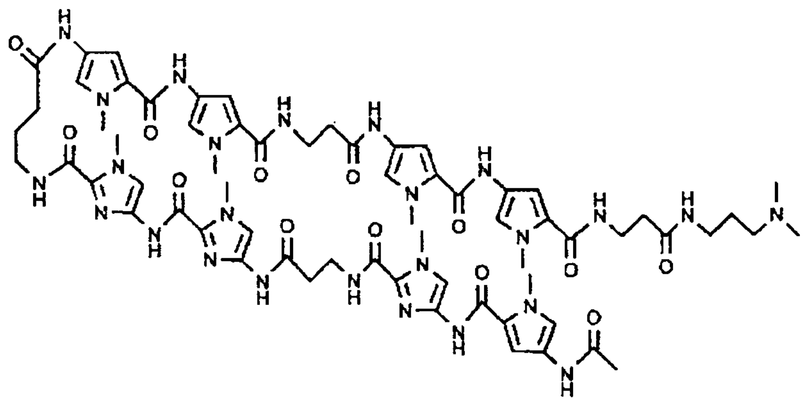

The monomers, reagents for synthesis, and column prepared in the same way as Example 1 were used to synthesize a polyamide by automated synthesis using a synthesizer and manual synthesis based on the acid chloride method. Synthesis was performed in the same way as Example 1 except that the acid chloride method was performed under the following conditions, and the resultant polypeptide was purified and recovered.
The result of HPLC after separation/purification is shown in Fig. 2 (0.1 % AcOH: $CH_3CN$ = 100:0 to 33.3:66.7, 20 min). After separation/purification, the resultant produce was freeze-dried, to thereby yield a target polyamide AcPyImβImImγPyPyβPyPyβDp (53.5 mg, 37.6%, ESI mass found; 1423.0, calcd for $[M+H]^+$; 1422.7).

Peptide synthesis (manual: acid chloride method)

In the nitrogen atmosphere, FmocPyCOOH (145 mg) was suspended in DCM (dichloromethane; manufactured by Wako Pure Chemicals Industries, Ltd., 2 mL) in an amount for one coupling. Oxalyl chloride (manufactured by Nacalai Tesque, Inc., 51.5 μL) was added dropwise to the suspension, and DMF (2 μL) was added thereto, followed by stirring at room temperature for 30 minutes. After the reaction, the solvent was distilled off under reduced pressure, to thereby yield an FmocPy acid chloride as a foam-like solid.
Pyridine (130 μL) was added to the swollen $H_2$NPyImPImImγPyPyβPyPy-Resin, and an FmocPy acid chloride dissolved in DCM (2 mL) was added thereto. The whole was shaken at room temperature for 20 minutes, and the resin was washed well with DMF, methanol, and methylene chloride.
In the same way as Example 1, separation/purification and freeze-drying were performed, to thereby yield a target polyamide AcPyImβImImγPyPyβPyPyβDp. The recovered product weight and yield of AcPyImβImImγPyPyβPyPyβDp are shown in Table 7.

[Example 13]

<Synthesis of AcPyImβImImImγPyPyβPyPyβDp by dimer method>

AcPyImβImImγPyPyβPyPyβDp (Formula 4) was synthesized by the dimer method as described in Example 11 using HCTU as a condensation-activator, and the yield was determined. The recovered product weight and yield are shown in Table 7.

[Comparative Example 7]

<Synthesis of AcPyImβImImγPyPyβPyPyβDp by acid anhydride method>

AcPyImβImImγPyPyβPyPyβDp (Formula 4) was synthesized by the acid anhydride method as described in Comparative Example 5 using HCTU as a condensation-activator, and the yield was determined. The recovered product weight and yield are shown in Table 7.

[Table 7]

| | Compound | Synthesis method | Recovered product weight | Yield |
|---|---|---|---|---|
| Example 12 | AcPyImβImImγPyPyβPy PyβDp | Acid chloride method | 53.5 mg | 37.6% |
| Example 13 | | Dimer method | 62.1 mg | 43.7% |
| Comparative Example 7 | | Acid anhydride method | 36.4 mg | 25.6% |

The results of Examples 12 and 13 and Comparative Example 7 reveal that the yields in the cases of the synthesis by the acid chloride method and dimer method using HCTU as a condensation-activator is higher (about 40.0%) than the yield in the case of the synthesis by the acid anhydride method.

Industrial Applicability

According to the synthesis method of the present invention, it is possible to rapidly and quantitatively synthesize a pyrrole-imidazole polyamide, which can be used for medical purposes or the like. In addition, it is possible to provide a synthesizer including a system based on the synthesis method, which is automated at a higher level and can produce a product with a high yield in a more stable manner.

**Claims**

1. A method of synthesizing a pyrrole-imidazole polyamide by a solid-phase synthesis method, comprising using as a condensation-activator HCTU (1-[bis(dimethylamino)methylene]-5-chloro-1H-benzotriazolium 3-oxide hexafluorophosphate).

2. A synthesis method according to Claim 1, comprising charging a material monomer in a solid form.

3. A synthesis method according to Claim 1 or 2, comprising forming a pyrrole bond or an imidazole bond to an N-terminal by an acid chloride method.

4. A synthesis method according to Claim 3, wherein the material monomer is a pyrrole acid chloride or an imidazole acid chloride.

5. A synthesis method according to Claim 1, comprising charging a pyrrole material and an imidazole material as a pyrrole-imidazole dimer based on a dimer method.

6. A synthesis method according to Claim 5, which is performed by automated synthesis.

7. A pyrrole-imidazole polyamide, which is represented by Formula 1.

[Formula 1]

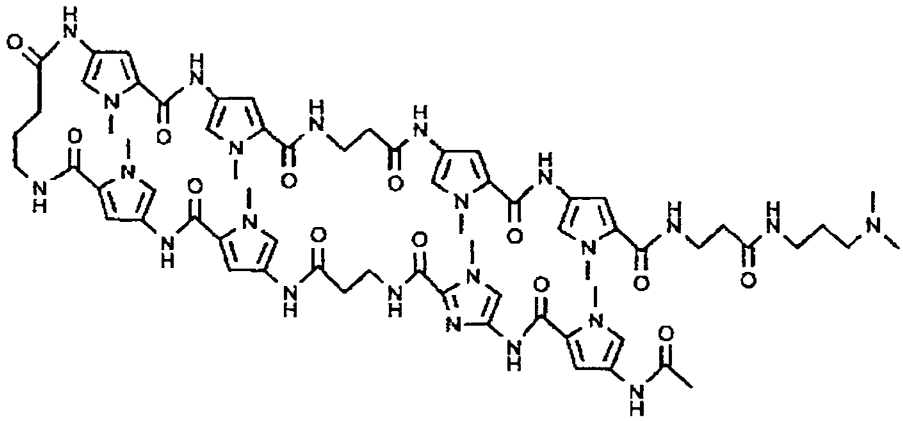

8. A pyrrole-imidazole polyamide, which is represented by Formula 2.

[Formula 2]

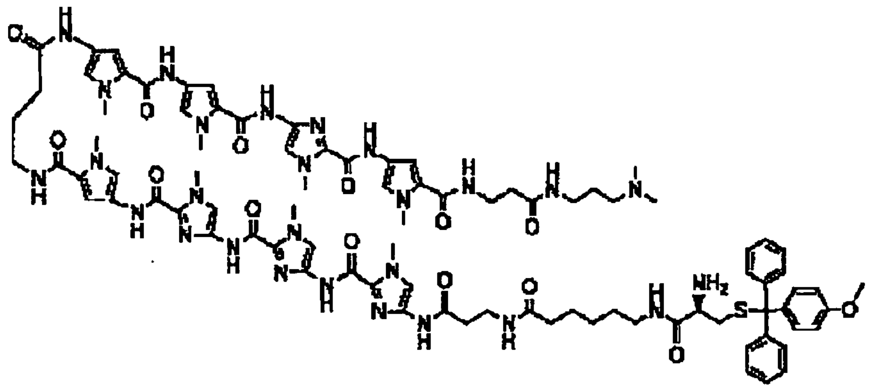

9. A pyrrole-imidazole polyamide, which is represented by Formula 3.

[Formula 3]

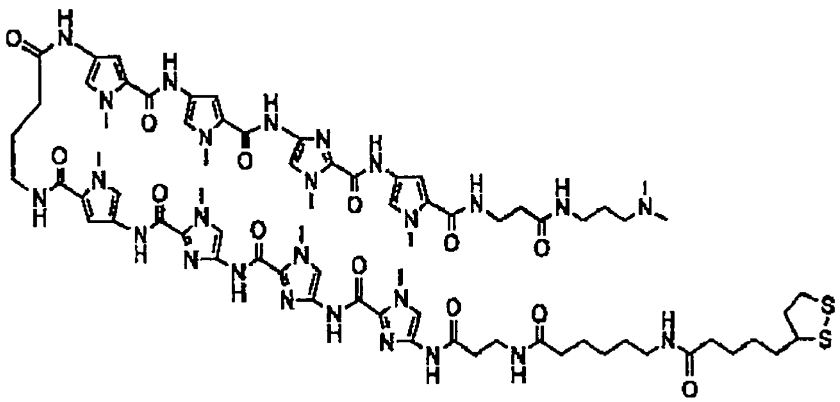

10. A pyrrole-imidazole polyamide, which is represented by Formula 4.

[Formula 4]

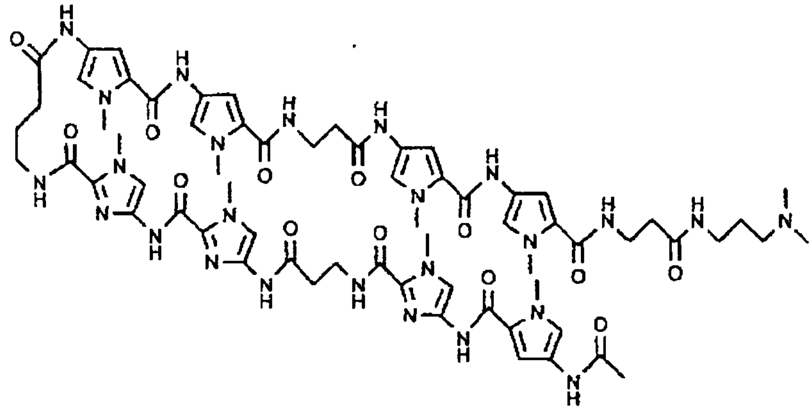

Fig. 1

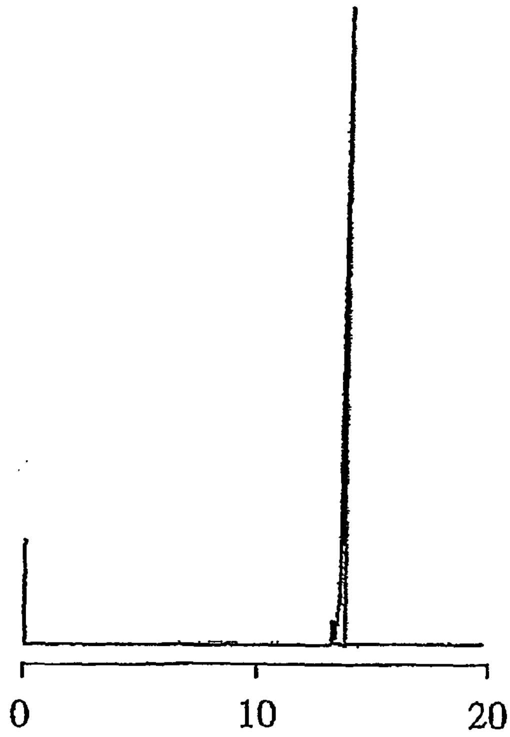


Fig. 2

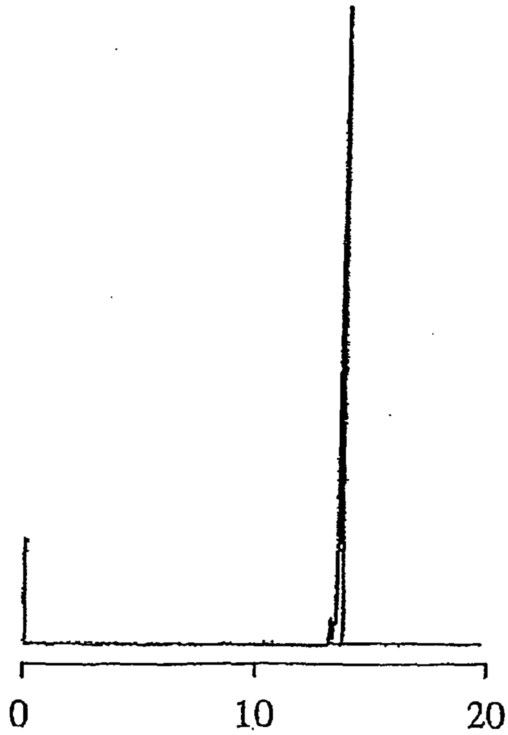

## INTERNATIONAL SEARCH REPORT

International application No.
PCT/JP2006/322658

A. CLASSIFICATION OF SUBJECT MATTER
*C07D403/14*(2006.01)i, *C07B61/00*(2006.01)i, *C07D409/14*(2006.01)i

According to International Patent Classification (IPC) or to both national classification and IPC

B. FIELDS SEARCHED

Minimum documentation searched (classification system followed by classification symbols)
C07D403/14, C07B61/00, C07D409/14

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y<br>A | WO 2003/000683 A1 (JAPAN SCI & TECHNOLOGY AGENCY),<br>03 January, 2003 (03.01.03),<br>Full text<br>& US 2004/171799 A1 & JP 2003-507086 A | 1-6<br>7-10 |
| Y<br>A | Marder O. et al., 'HCTU and TCTU. New coupling reagents: Development and industrial aspects', Chimica Oggi, 20(7/8), pp.37-41, 2002 | 1-6<br>7-10 |
| A | WO 2005/023248 A1 (UNIV NIPPON),<br>17 March, 2005 (17.03.05),<br>Full text<br>& DE 112004001603 T5 | 1-10 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 February, 2007 (13.02.07) | 27 February, 2007 (27.02.07) |
| Name and mailing address of the ISA/<br>Japanese Patent Office<br>Facsimile No. | Authorized officer<br><br>Telephone No. |

Form PCT/ISA/210 (second sheet) (April 2005)

**INTERNATIONAL SEARCH REPORT**

International application No.
PCT/JP2006/322658

C (Continuation). DOCUMENTS CONSIDERED TO BE RELEVANT

| Category* | Citation of document, with indication, where appropriate, of the relevant passages | Relevant to claim No. |
|---|---|---|
| A | WO 2003/076412 A1 (JAPAN SCI & TECHNOLOGY AGENCY),<br>18 September, 2003 (18.09.03),<br>Full text<br>& JP 2003-261541 A & EP 1491534 A1<br>& US 2005/176647 A1 | 1-10 |
| A | WO 2005/087762 A1 (TMRC CO., LTD.),<br>22 September, 2005 (22.09.05),<br>Full text<br>(Family: none) | 1-10 |
| A | JP 2001-500134 A (ZEALAND PHARM AS),<br>09 January, 2001 (09.01.01),<br>Full text<br>& WO 98/11125 A1 & AU 9741993 A<br>& EP 929567 A1 & IL 128829 A | 1-10 |
| A | JP 2002-515063 A (CALIFORNIA INST OF TECHNOLOGY),<br>21 May, 2002 (21.05.02),<br>Full text<br>& WO 98/49142 A1 & AU 9871040 A<br>& EP 986539 A1 | 1-10 |
| A | JP 2001-513759 A (CALIFORNIA INST OF TECHNOLOGY),<br>04 September, 2001 (04.09.01),<br>Full text<br>& WO 98/37066 A1 & AU 9864334 A<br>& EP 968186 A1 | 1-10 |
| P,A | JP 2006-022063 A (UNIV NIPPON),<br>26 January, 2006 (26.01.06),<br>Full text<br>(Family: none) | 1-10 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2003000683 A
- JP 2001500134 A